# EUROPÄISCHE PATENTSCHRIFT

(11) **EP 0 687 304 B1**
(45) Veröffentlichungstag und Bekanntmachung des Hinweises auf die Patenterteilung: **15.10.1997**
(21) Anmeldenummer: 94906216.0
(22) Anmeldetag: 03.02.1994
(51) Int. Cl.: C12N 15/86, C12Q 1/68, C12Q 1/02, G01N 33/50, C12N 15/63, C12Q 1/66

(54) **IN-VITRO-TEST ZUR DETEKTION VON PROTEINKINASE C INHIBITOREN**
METHOD OF DETECTING PROTEIN KINASE C INHIBITORS
TEST IN VITRO PERMETTANT DE DETECTER DES INHIBITEURS DE LA PROTEINE-KINASE C

(30) Priorität: 03.03.1993 DE 4306547
(43) Veröffentlichungstag der Anmeldung: 20.12.1995
(73) Patentinhaber: GSF-Forschungszentrum für Umwelt und Gesundheit, GmbH, D-85758 Oberschleissheim (DE)
(72) Erfinder: BORNKAMM, Georg, D-81243 München (DE); POLACK, Axel, D-80798 München (DE)
(86) Internationale Anmeldenummer: EP9400314
(87) Internationale Veröffentlichungsnummer: WO9420630

(56) Entgegenhaltungen:
- EP-A- 0 370 813
- EP-A- 0 516 443
- WO-A-89/07654
- WO-A-93/04179
- INTERNATIONAL J. CANCER, vol. 50, no. 4, 20. Februar 1992, INT. J. CANCER, GENEVA,CH, Seiten 611-616; A. POLACK ET AL: 'Short-term assay for detection of conditional cancerogens I. Construction of DR-CAT Raji cells and some of their characteristics as tester cells'
- INTERNATIONAL J. CANCER, vol. 40, no. 6, 15. Dezember 1987, INT. J. CANCER, GENEVA,CH, Seiten 846-849; J. LAZDINS ET AL: 'TPA induction of Epstein-Barr virus early antigens in Raji cells is blocked by selective protein kinase-C inhibitors'
- CARCINOGENESIS, vol. 14, Nr. 8, August 1993, OXFORD UNIVERSITY PRESS, OXFORD,GB, Seiten 1573-1578; G. BOUVIER ET AL: 'Validation of two test systems for detecting tumor promoters and EBV inducers: comparative responses of several agents in DR-CAT Raji cells and in human granulocytes'

## Beschreibung

Die Erfindung betrifft einen In-vitro-Test zur Detektion von Proteinkinase C Inhibitoren.

Die Wirkung von bestimmten Tumorpromotoren auf Zellen oder Organismen werden über die Aktivierung der Proteinkinase C (PKC) vermittelt. Tumorpromotoren sind Substanzen, die in Kombination mit für sich nicht tumorigenen Dosen von Solitärkarzinogenen die Entwicklung von Tumoren bzw. Karzinomen induzieren. Solitärkarzinogene sind Substanzen, die (im Tierversuch) für sich allein appliziert, dosisabhängig Tumoren hervorrufen. Die alleinige Einwirkung von Tumorpromotoren dagegen erzeugt im Sinne ihrer Definition keinen Krebs, sie fördert aber in erheblichem Maße in solchen Organen, die durch exogene Einwirkung eines Solitärkarzinogens initiiert sind, die Tumorentstehung.

Ein von zur-Hausen, H., Bornkamm, G. W., Schmidt, R., und Hecker, E.: Tumor initiators and promoters in the induction of Epstein-Barr virus. in: Proceedings of the National Academic Science (U.S.A.) Band 76, Seiten 782 - 785 (1979) beschriebener Ansatz sieht vor, die Epstein-Barr Virus (EBV) induzierende Eigenschaft von Tumorpromotoren (und anderen Substanzen) mittels Immunfluoreszenzdarstellung, EBV kodierter Antigene (sog. frühe Antigene) zu ermitteln. Diese Technik setzt jedoch virologisch und serologisch geschultes Personal voraus. Darüber hinaus ist dieses Verfahren zeitaufwendig und aufgrund der subjektiven Bewertung von immunofluoreszenzgefärbten Zellen nicht exakt standardisierbar.

Mit Hilfe dieses Systems war es möglich zu zeigen, daß die Aktivierung der PKC durch die Tumorpromotoren ein entscheidender Schritt bei der intrazellulären Signalweitergabe darstellt (Lazsins, J., ompetta, C., Grimaldi, S., Barile, G., Venanzoni, M., Farti, L., and Faggioni, A. (1987). TPA induction of Epstein-Barr virus early antigens in Raji cells is blocked by selection protein kinase-C inhibitors. Int. J. Cancer 40, 846 - 849.

Das System der Tumorpromotor vermittelten EBV Induktion erlaubt daher die Erfassung von PKC Inhibitoren. Aufgrund der oben genannten technische Limitierungen erlaubt dieses System (Immunfluoreszenztechnik) unter anderem keinen hohen Testprobendurchsatz.

Aus Polack, A. et al. (1992), Int. J. Cancer: 50, 611-616, sind EBV haltige Zellen bekannt, die zwar ein pHEBO-DR-CAT Konstrukt, jedoch keine Glühwürmchenluziferase enthalten.

Des weiteren ist aus Lazdins, J. et al. (1987), Int, J. Cancer: 40, 846-849 bekannt, daß TPA Induktion von EBV frühen Antigenen in Raji-Zellen unter anderem durch PVK Inhibitoren blockiert werden kann. Der Regulationsmechanismus ist jedoch nicht eindeutig und auf andere Mechanismen wird hingewiesen.

Inhibitoren der PKC stellen in naher Zukunft sehr wahrscheinlich therapeutisch wertvolle Substanzen dar. Die Aktivierung der PKC's scheint bei der Entstehung von einer Vielzahl von pathologischen Zuständen entscheidend beteiligt zu sein (Akinaga, S., Gomi, K., Morimoto, M., Tamaoki, T., and Okabe, M. (1991). Antitumor activity of UCN-01, a selective inhibitor of protein kinase C, in murine and human tumor models. Cancer Res. 51, 4888-4892).

Dies gilt insbesondere bei der Entstehung einer akuten Entzündungen bzw. der Aktivierung von Viren aus dem Zustand der Latenz (z. B. Humanes Immundefizienzvirus oder EBV).

Aufgabe der Erfindung ist es nun, einen einfach durchführbaren quantitativen Test der e. g. Art zur Verfügung zu stellen.

Diese Aufgabe wird durch den Patentanspruch 1 gelöst. Die übrigen Ansprüche beschreiben vorteilhafte Ausgestaltungen der Erfindung.

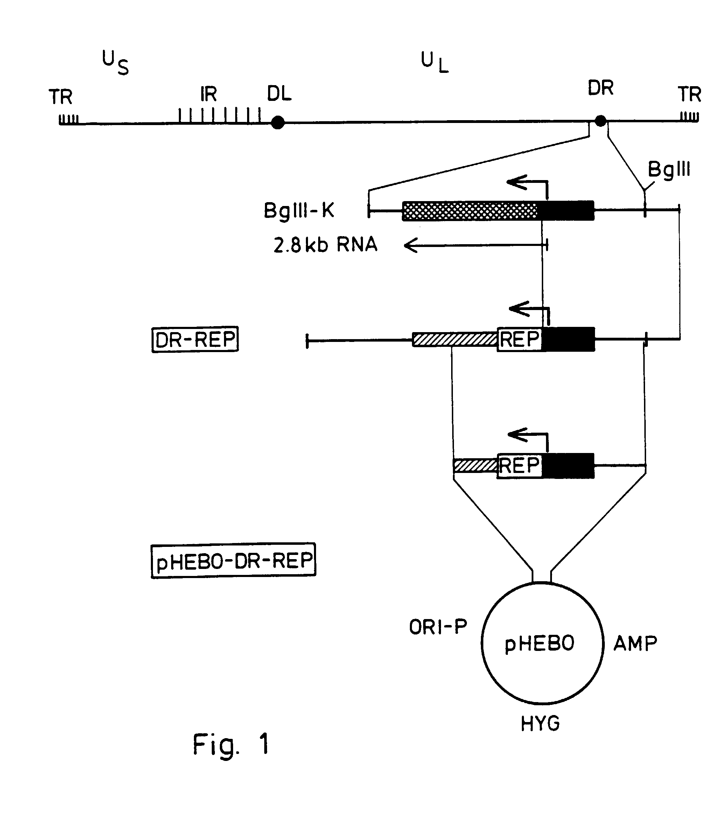

Die Erfindung wird im folgenden anhand zweier Beispiele und der Figuren 1 bis 3 näher erläutert.

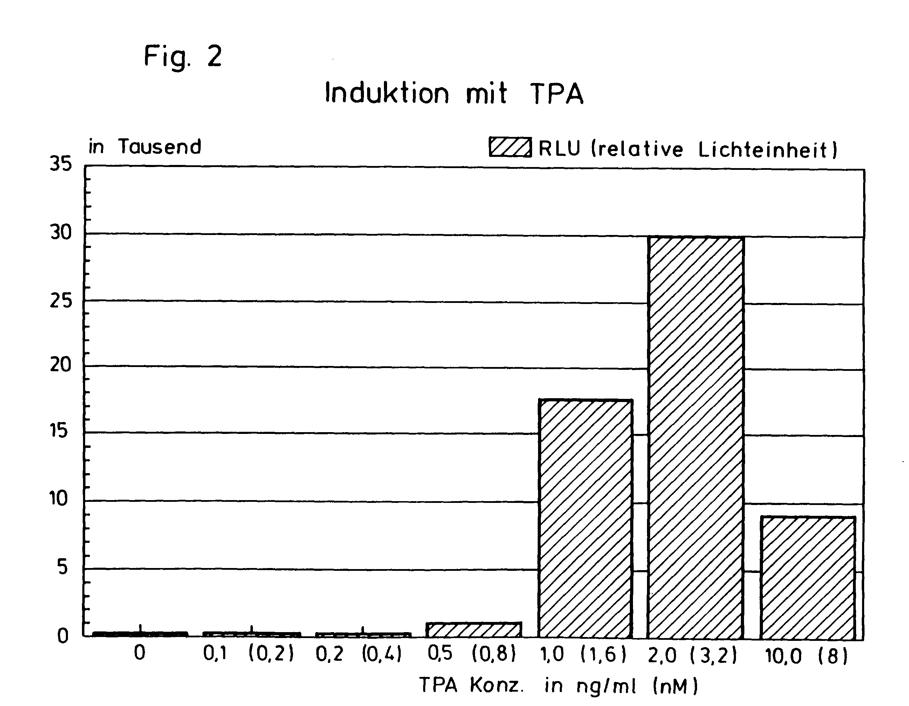

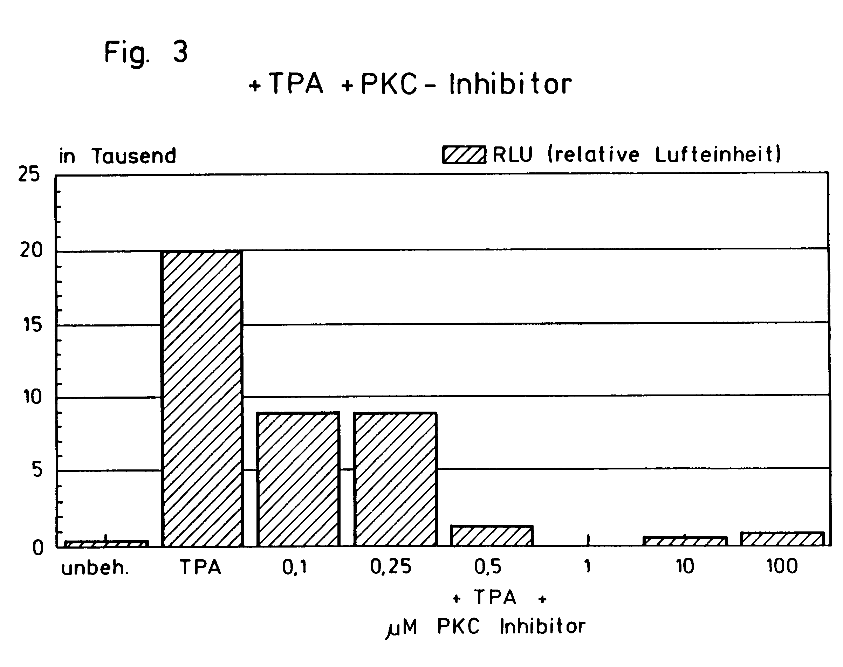

Die Fig. 1 zeigt schematisch den Aufbau des DNA Konstrukts, während die Figuren 2 und 3 die Wirkung eines Tumorpromotors, sowie die Wirkung eines PKC Inhibitors zeigen.

Die Basen-Sequenz und die Kartierung eines DNA Konstruktes findet sich in der Tabelle 1.

Die Erfindung basiert auf der Beobachtung, daß verschiedene Klassen von Tumorpromotoren, wie z. B. Diterpenester, Indolalkaloide oder Polyacetate, Teleocidin, transforming growth factor β (TGFß) in Epstein-Barr Virus (EBV) haltigen lymphatischen Zellen einen lytischen oder abortiven Viruszyklus induzieren. Der Test zur Detektion von PKC Inhibitoren kann mit jeder dieser Verbindungen durchgeführt werden. Im EBV Genom gibt es eine große Anzahl von Genen, die im Zustand der Latenz nicht exprimiert sind und erst bei Induktion eines lytischen oder abortiven Zyklus induziert werden. Die für diese Aktivierung verantwortliche Kontrollregion des besonders stark induzierten DR-Gens (DL oder andere sind möglich) wurde vor den kodierenden Bereich eines sogenannten Reportergens mit den Methoden der Molekularbiologie kloniert. Der hierfür verwandte Vektor (pHEBO) trägt den episomalen Repliaktionsursprung von EBV und zwei Resistenzgene, von denen das eine die Selektion transifzierter eukaryonter Zellen mit Hygromyzin, und das Andere die Selektion in Prokaryonten erlaubt. Dieser Vektor ist beschrieben von Sugden, B., Marsh, K., and Yates J.A. vector taht replicates as a plasmid and can be efficiently selected in B-lymphoblasts transformed by Epstein virus. in: Molecular and Cellular Biology, Band 5, Seiten 410 bis 413 (1985). Das so entstandene Konstrukt (siehe auch Fig. 1) wurde stabil in eine EBV haltige Zellinie (Raji) eingeschleust.

Raji-Zellen wurden gewählt, da aufgrund von Deletionen im Raji-EBV-Genom kein infektiöses Virus gebildet werden kann und weil die sogenannte Spontaninduktionsrate in diesen Zellen sehr gering ist. Als Zielzellen für die stabile Transfektion mit dem oben beschriebenen Plasmid kommen alle EBV positiven Zellinien in Betracht, die sich durch chemische Induktoren induzieren lassen (z. B. die Zellinie P3HR1 oder Akata). Das Translationsprodukt des Reportergens kann auf relativ einfache Weise über eine Enzymreaktion in Extrakten von diesen Zellen quantitativ nachgewiesen werden. Die Menge an nachweisbarem Reportergenprodukt korreliert quantitativ mit dem Aktivierungszustand des EBV-Genoms.

Aufgrund der vollständigen Unterdrückung der Tumorpromotoren vermittelten EBV Induktion durch die Anwendung von PKC Inhibitoren kann dieses System als einfach und schnell durchführbarer Suchtest zur Identifikation von PKC Inhibitoren eingesetzt werden.

Es wurden die Reportergene (REP) Chlorampenicolacetyltransferase (CAT) und die Glühwürmchen-Luziferase (LUC) getestet.

Weitere verwendbare Reportergene sind: die β-Galaktosidase, das β-Globin-Gen, Hepatitis B kodierte Antigene o. ä.

Das hier beschriebene Testsystem unterscheidet sich von den bisher bekannten vor allem durch eine vereinfachte Durchführbarkeit und eine entscheidende Verbesserung der quantitativen Auswertung der Ergebnisse. Bisher floß in jedes Meßergebnis die subjektive Bewertung von immunfluoreszenzgefärbten Zellen ein. Insbesondere beinhaltet die Angabe, ein bestimmter Prozentsatz von Zellen zeige eine positive Immunfluoreszenz für frühe EBV-Antigene, eine subjektive Festsetzung eines Schwellenwertes. Darüber hinaus läßt sich auf diese Weise die absolute Proteinmenge an frühen Antigenen, die in der einzelnen Zelle entstanden sind, nicht bestimmen. Dies erschwerte in der Vergangenheit eine Standardisierung erheblich.

Die Menge an entstandenem Reportergenprodukt läßt sich dagegen exakt und reproduzierbar in einer vorgebenen Anzahl von Zellen bestimmen. Ein weiterer Vorteil dieses Systems ist durch den klonalen Ursprung der stabil transfizierten Raji-DR-REP-Linie gegeben. Es ist bekannt, daß im Rahmen der langen in-vitro-Kultivation der Raji-Zellinie Subpopulationen mit unterschiedlichem biologischen Verhalten entstanden sind. Darüber hinaus zeichnet sich dieser Test durch eine höhere Sensitivität und eine verbesserte Dosis-Wirkungsbeziehung aus.

Die Figur 1 zeigt als Diagramm die Konstruktion der DNA-Klone (DNA Konstrukte) DR-REP und pHEBO-DR-REP.

### REP steht für Reportergen.

Die oberste Zeile der Zeichnung stellt das Genom des EBV in linearer Form dar (terminale repetitionen, TR; interne Repetionen, IR; duplizierte Region links und rechts, DL und DR; lange und kurze einheitliche Region, U_{L} und U_{S}). In der nächsten Zeile ist das BglII-Restriktionsfragment (BglII-K) (Polack et al. A complete set of overlapping cosmid clones of M-ABA virus derived from nasopharyngeal carcinoma and its similarity to other Epstein-Barr virus isolates. in: Gene, Band 27, Seiten 279 - 288, 1984), das das DR-Gen trägt, vergrößert dargestellt. Der Pfeil in der nächsten Zeile entspricht der von dieser Region kodierten 2,8 kb großen Boten-RNS.

Die Figur 2 zeigt die Induktion des DR-LUC Konstruktes durch TPA. Stabil mit dem Konstrukt pHEBO-DR-LUC transfizierte Raji-Zellen wurden mit den angegebenen Konzentrationen von TPA behandelt. In den Zellen wurde nach 48-stündiger Inkubation in Abhängigkeit von der Konzentration an TPA Luziferase gebildet. Die höchste Menge an Luziferase wurde bei einer TPA Konzentration von 2,0 ng/ml beobachtet, die daher dem in Figur 3 gezeigten Experiment zugrunde gelegt wurde.

Figur 3 zeigt die Wirkung des PKC Inhibitors (Bisindolylmaleimide GF 109203X, beschrieben von: Toullec, D., Pianetti, P., Coste, H., Bellevergue, P., Grand-Perret, T., Ajakane, M., Baudet, V., Boissin, P., Boursier, E., Loriolle, F., and et al (1991). The bisindolylmaleimide GF 109203X is a potent and selective inhibitor of protein kinase C. J. Biol. Chem. 266, 15771 - 15781) auf die TPA ausgelöste EBV Induktion. Die TPA-Konzentration beträgt dabei 2,0 mg/ml.

Ein Beispiel für einen In-vitro Test besteht aus folgenden Verfahrensschritten:
1. Züchtung von mindestens 0,1 bis 1,0 x 10⁴ mit pHEBO-DR-LUC-transfizierten Raji-Zellen pro experimentellem Ansatz. Zellkulturbedingungen:
   Zellkulturmedium: RPMI 1640 (Gibco) mit 10 % foetalem Kälberserum, 300 µg/ml Hygromycin (Calbiochem), 100 U/ml Penizillin, 280 µg/ml L-Glutamin, 37° C, 5 - 6 % CO₂.
2. Inkubation dieser Zellen mit einer entsprechenden Menge zu testender Substanz und einer geeigneten Menge Tumorpromotor (hier 2 ng/ml TPA) für 48 Stunden (= Standardansatz; <20 h bis zu mehreren Tagen sind ebenfalls möglich) bei 37° C und 6 % CO₂.
3. Gewinnung der Zellen durch Zentrifugation.
4. Resuspension der Zellen in Lysepuffer (100 mM Kaliumphosphat pH 7.8, 1 mM DTT).
5. Aufschluß der Zellen zur Gewinnung des gebildeten Enzyms mittels Ultraschallbehandlung, drei Zyklen Frieren/Tauen (3 Minuten im CO₂/Äthanol-Bad/3 Minuten Tauen bei 30° C) oder Detergens-Behandlung.
6. Klärung des Zell-Lysats durch Zentrifugation.
7. Bestimmung der Proteinkonzentration
8. Inkubation von z. B. 20 µl Lysat (Menge richtet sich nach der Proteinmenge) 100 µl Testpuffer (25 mM Glycylglycine, pH 7.8, 5 mM ATP, 15 mM MgSO₄) Injektion von 100 µl Luciferinlösung (1 mM Luciferin in 0,5 M Tris-HCl (pH 7.8))
9. sofortige Messung des emittierten Lichts in einem Luminometer (z. B. Lumat von Berthold).
10.Kontrollen: unbehandelte und nur mit TPA behandelte Raji-DR-LUC-Zellen.

Die Menge der nachgewiesenen Enzyme ist ein Maß für die Eigenschaft der untersuchten Substanzen, die EBV Induktion durch TPA zu supprimieren und läßt somit einen Rückschluß zu auf die mögliche PKC inhibierende Eigenschaft dieser Substanz.

Die Menge an entstandenem Enzym kann im Luziferase-Test über mehrere Log-Stufen ohne zusätzliche Verdünnungsreihen quantitativ erfaßt werden.

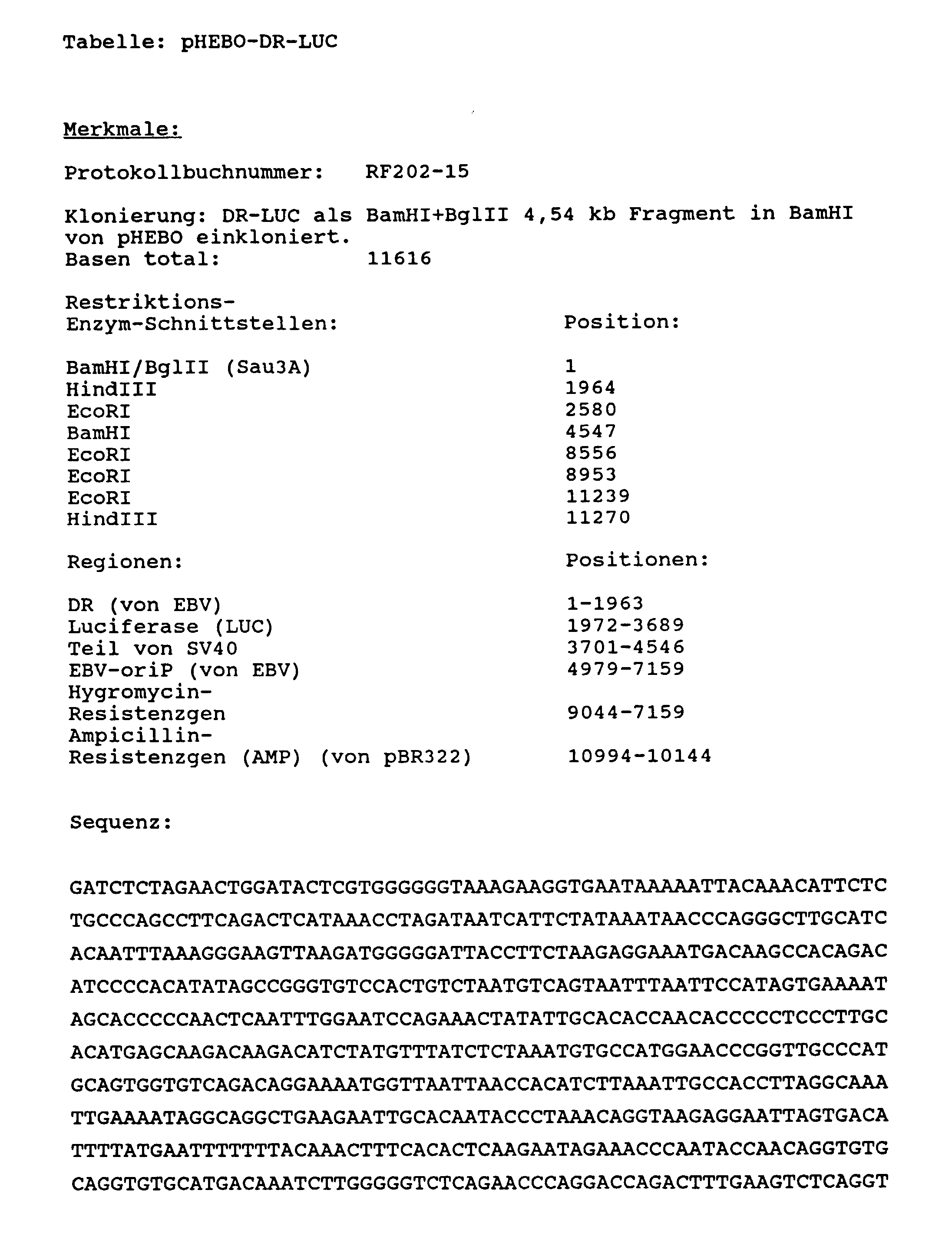

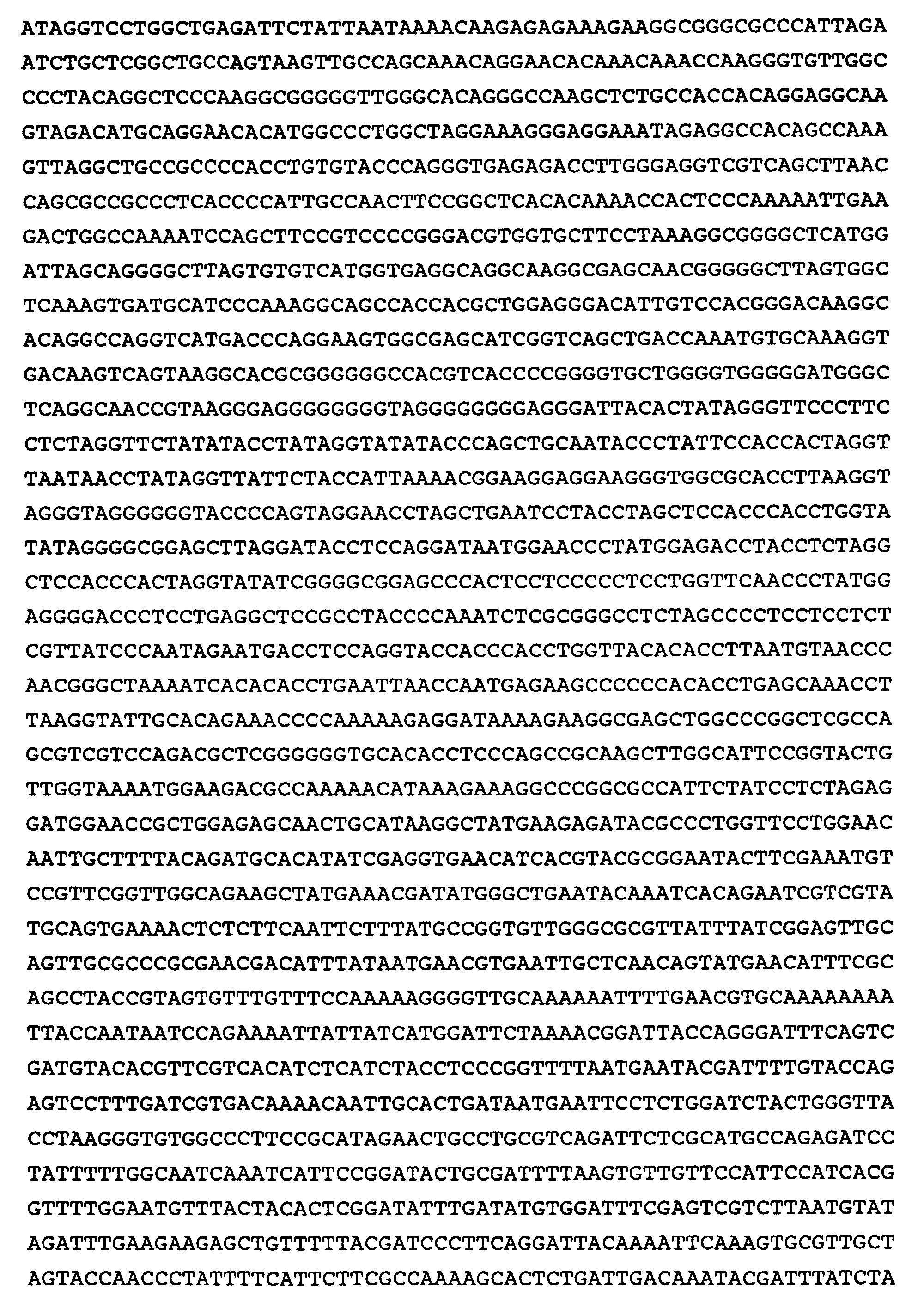

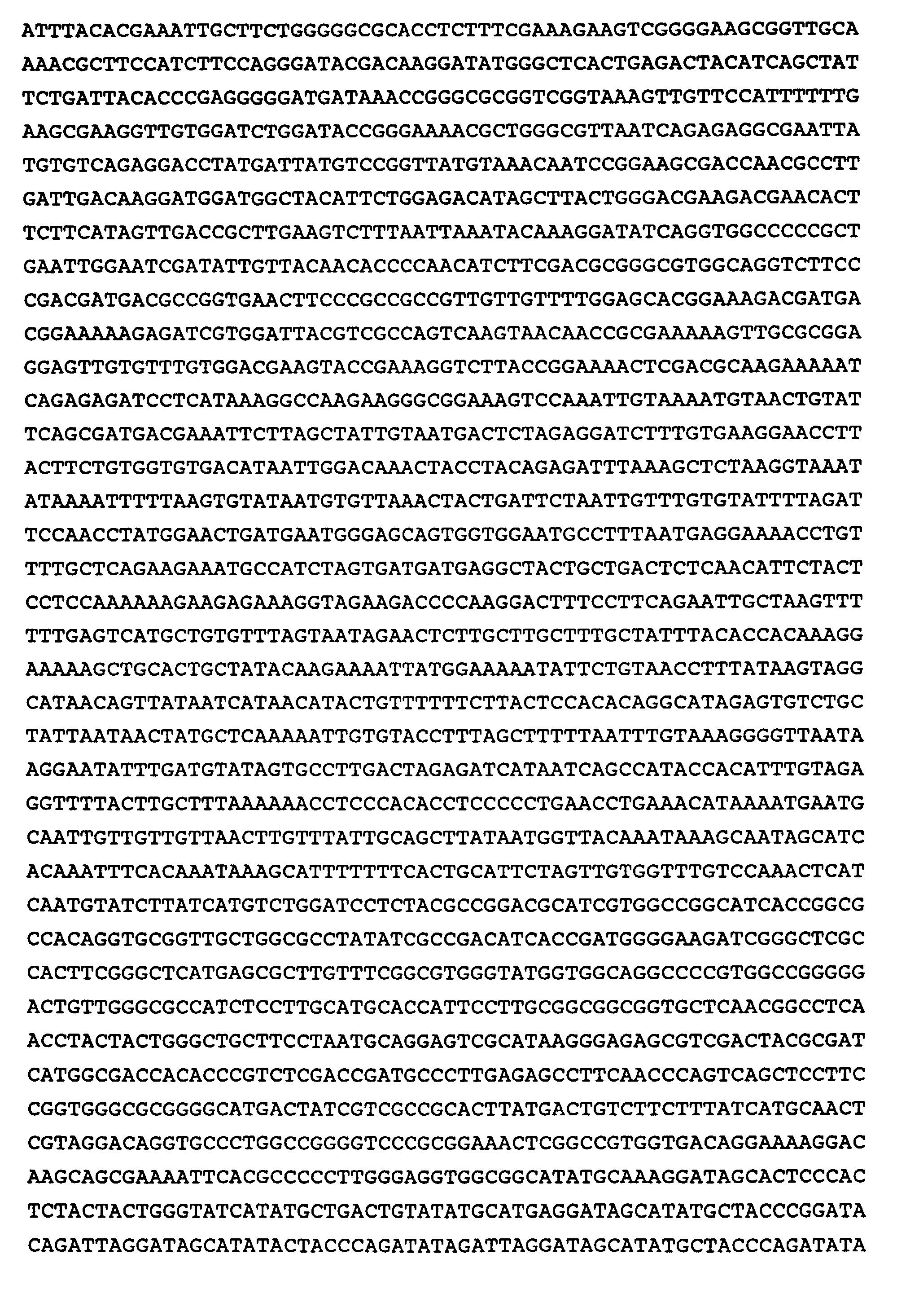

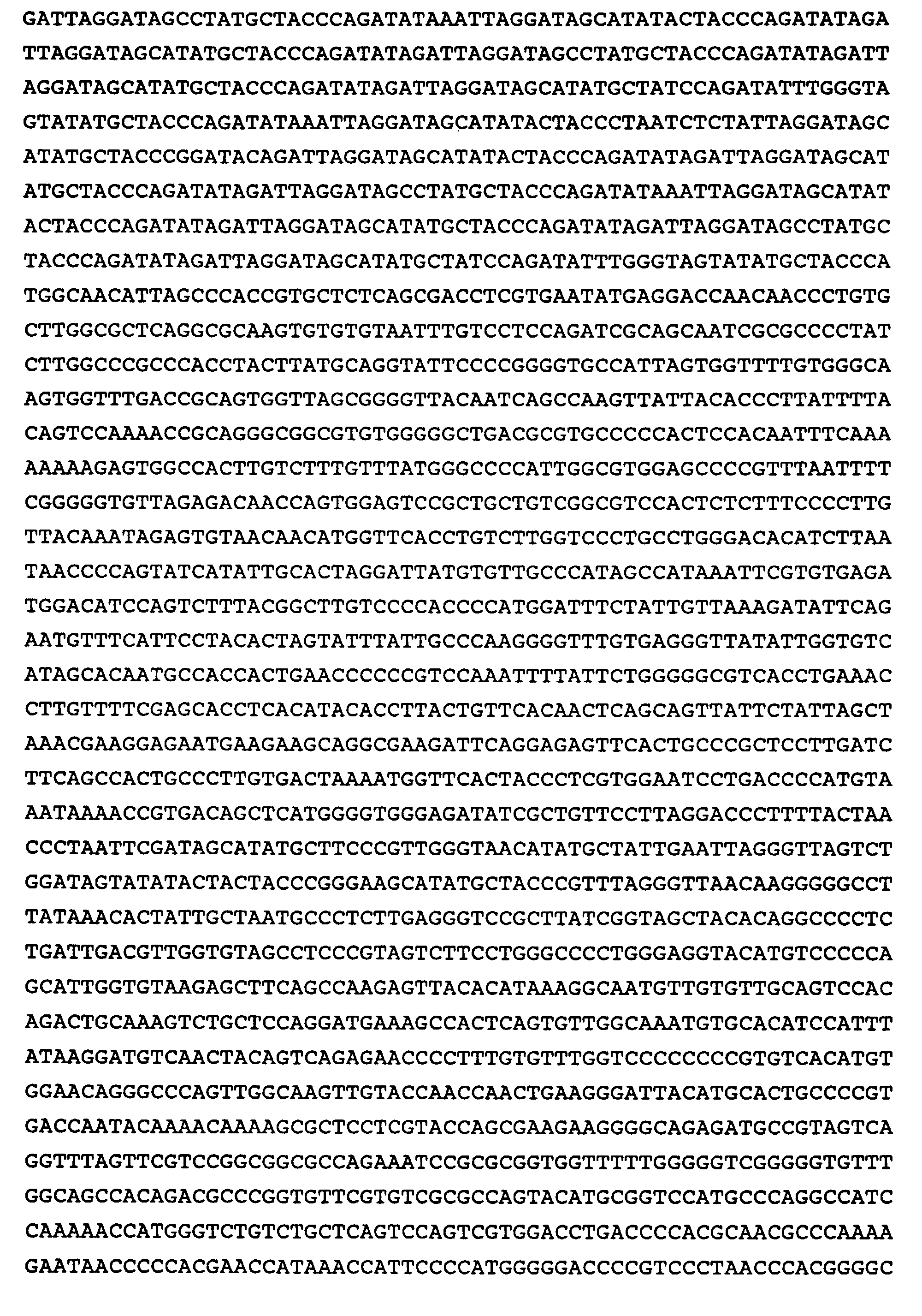

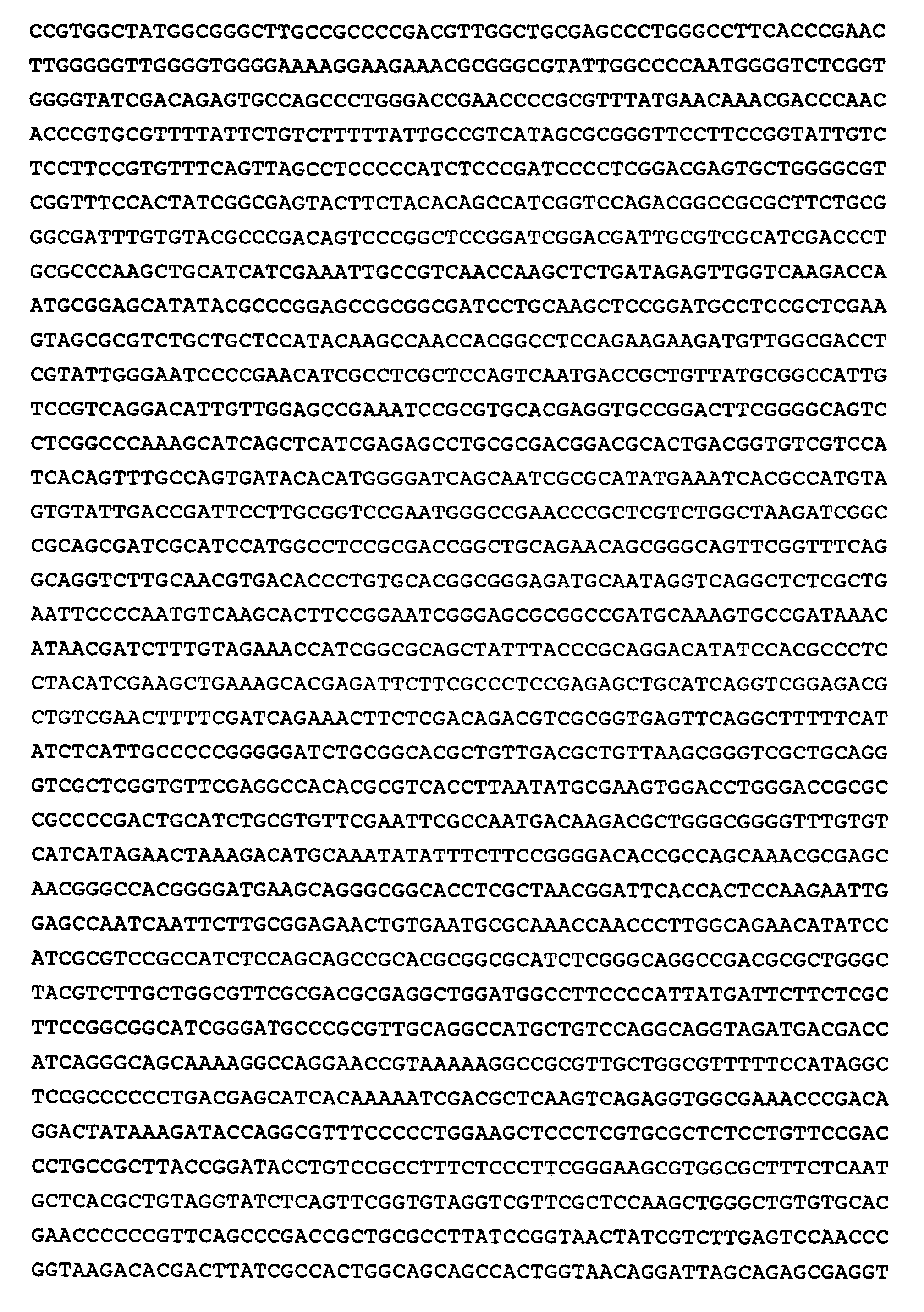

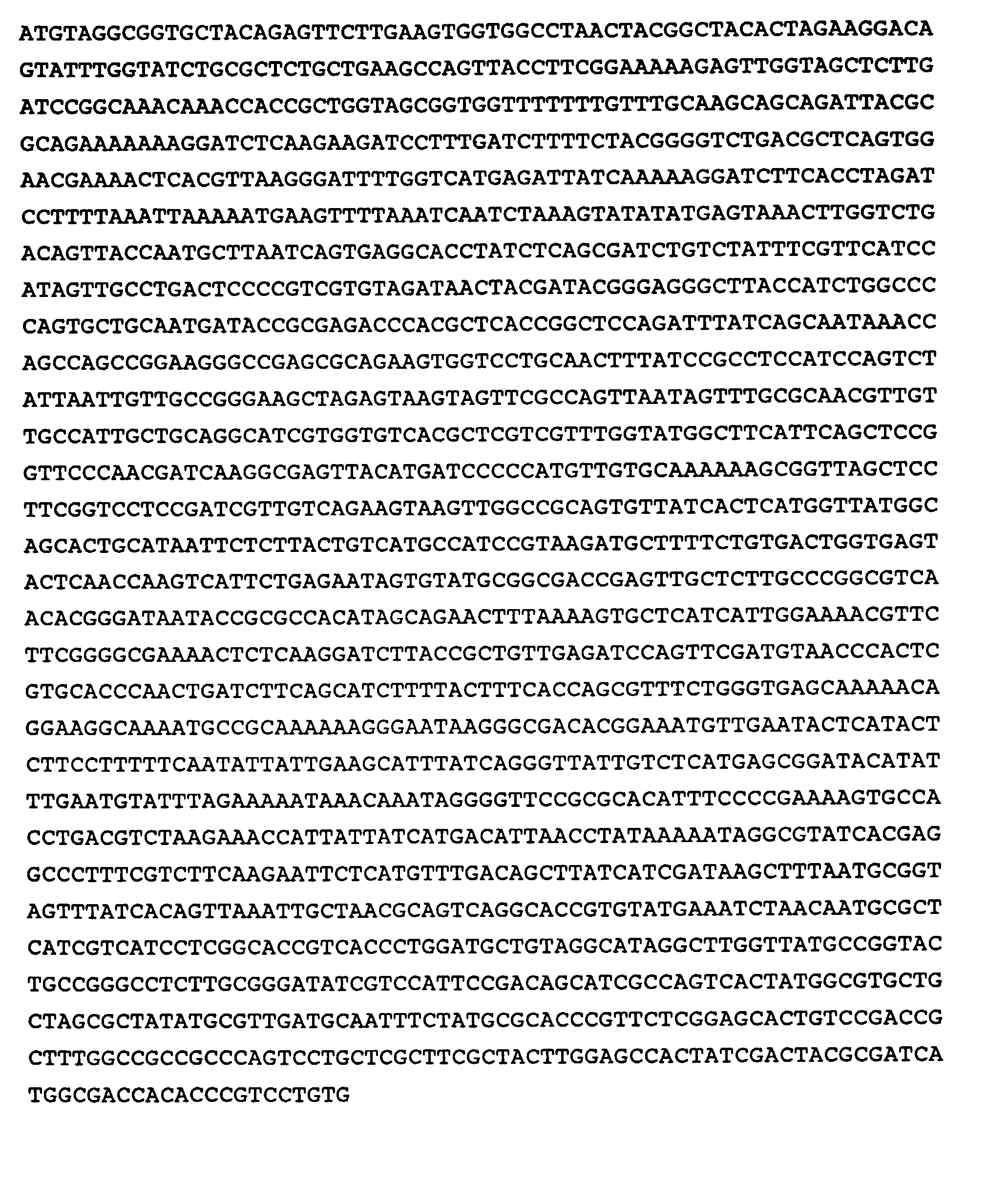

## Patentansprüche

1. In-vitro-Test zur Detektion von Proteinkinase C Inhibitoren gekennzeichnet durch folgende Verfahrensschritte:
a) EBV-haltige Zellen, welche ein DNA Konstrukt enthalten, welches aus einer Kontrollregion für die Aktivierung eines besonders stark induzierten Gens des Epstein-Barr-Virus (EBV) und Glühwürmzchenluziferase (LUC) als Reportergen (REP) besteht, werden mit einem PKC aktivierenden Tumorpromotor und der zu untersuchenden Substanz in Kontakt gebracht, als Kontrolle dienen die unbehandelten Zellen sowie die nur mit Tumorpromotor behandelten Zellen
b) dann wird die Mischung ca. 1 bis 3 Tage bei 37° C in CO₂-Atmosphäre gehalten,
c) schließlich wird die Menge vom entstandenen Reportergenprodukt bestimmt.

2. In-vitro-Test nach Anspruch 1, dadurch gekennzeichnet, daß als EBV-haltige Zellen Raji-Zellen verwendet werden.

3. In-vitro-Test nach Anspruch 1 oder 2, dadurch gekennzeichnet, daß als PKC aktivierender Tumorpromotor TPA verwendet wird.

4. In-vitro-Test nach einem der Ansprüche 1 bis 3, dadurch gekennzeichnet, daß ein DNA-Konstrukt verwendet wird, daß zusätzlich ein Resistenzgen R1 für die Selektion transfizierter eukaryonter Zellen und ein Resistenzgen R2 für die Selektion in procaryonten Zellen enthält.

5. In-vitro-Test nach einem der Ansprüche 1 bis 4, dadurch gekennzeichnet, daß der stark induzierte Promotor der DR-Promotor ist.

6. In-vitro-Test nach einem der Ansprüche 1 bis 5, dadurch gegennzeichnet, daß R1 ein Hygromyzin-Resistenzgen ist.

7. In-vitro-Test nach einem der Ansprüche 1 bis 6, dadurch gekennzeichnet, daß R2 ein Ampicillin-Resistenzgen ist.

## Claims

1. In vitro test for detecting protein kinase C inhibitors, characterised by the following method steps:
a) EBV-containing cells, which contain a DNA construct comprising a control region for the activation of a particularly strongly induced gene of the Epstein-Barr virus (EBV) and glowworm luciferase (LUC) as the reporter gene (REP), are brought into contact with a PKC-activating tumour promotor and the substance to be investigated, the untreated cells and the cells treated only with the tumour promotor serving as the control;
b) then the mixture is kept at 37° C in a CO₂ atmosphere for approx. 1 to 3 days; and
c) finally the quantity of resultant reporter gene product is determined.

2. In vitro test according to claim 1, characterised in that Raji cells are used as the EBV-containing cells.

3. In vitro test according to claim 1 or 2, characterised in that TPA is used as the PKC-activating tumour promotor.

4. In vitro test according to one of claims 1 to 3, characterised in that a DNA construct is used, which additionally contains a resistance gene R1 for the selection of transfixed eucaryotic cells and a resistance gene R2 for the selection in procaryotic cells.

5. In vitro test according to one of claims 1 to 4, characterised in that the strongly induced promotor is the DR promotor.

6. In vitro test according to one of claims 1 to 5, characterised in that R1 is a hygromyzin resistance gene.

7. In vitro test according to one of claims 1 to 6, characterised in that R2 is an ampicillin resistance gene.

## Revendications

1. Test « in vitro » de détection des inhibiteurs de protéine-Kinase C,
caractérisé par les étapes de procédé suivantes :
a) les cellules contenant EBV qui renferment un produit de construction d'ADN qui consiste en une région de contrôle pour l'activation d'un gène particulièrement fortement induit du virus d'Epstein-Barr (EBV) et en luciférase de ver luisant (LUC) comme gène rapporteur (REP) sont mises en contact avec un promoteur de tumeur activateur de PKC et avec la substance à étudier, et les cellules non traitées servent comme témoins ainsi que les cellules traitées seulement avec le promoteur de tumeur.
b) ensuite on maintient le mélange environ 1 à 3 jours à 37°C en atmosphère de CO₂.
c) finalement on détermine la quantité de produit de gène rapporteur formé.

2. Test « in vitro » selon la revendication 1,
caractérisé en ce qu'
on utilise comme cellules contenant EBV, des cellules Raji.

3. Test « in vitro » selon la revendication 1 ou la revendication 2,
caractérisé en ce qu'
on utilise comme promoteur de tumeur activateur de PKC, du TPA.

4. Test « in vitro » selon l'une des revendications 1 à 3,
caractérisé en ce qu'
on utilise un produit de construction d'ADN qui contient en supplément un gène de résistance R1 pour la sélection de cellules eucaryotes transfectées et un gène de résistance R2 pour la sélection dans des cellules procaryotes.

5. Test « in vitro » selon l'une des revendications 1 à 4
caractérisé en ce que
le promoteur fortement induit est le promoteur DR.

6. Test « in vitro » selon l'une des revendications 1 à 5,
caractérisé en ce que
R1 est un gène de résistance à l'hygromycine.

7. Test « in vitro » selon l'une des revendications 1 à 6,
caractérisé en ce que
R2 est un gène de résistance à l'ampicilline.
